(19) **Europäisches Patentamt / European Patent Office / Office européen des brevets**

(11) **EP 3 234 185 B1**

(12) # EUROPEAN PATENT SPECIFICATION

(45) Date of publication and mention
of the grant of the patent:
**08.04.2020 Bulletin 2020/15**

(21) Application number: **15817217.1**

(22) Date of filing: **17.12.2015**

(51) Int Cl.:
***C12Q 1/6883*** (2018.01)

(86) International application number:
**PCT/EP2015/080183**

(87) International publication number:
**WO 2016/097118 (23.06.2016 Gazette 2016/25)**

(54) **METHOD FOR PREDICTING RESPONSE TO CONTINUOUS POSITIVE AIR PRESSURE TREATMENT**

VERFAHREN ZUR VORHERSAGE DER REAKTION AUF EINE KONTINUIERLICHE POSITIVE ATEMWEGDRUCKBEHANDLUNG

PROCÉDÉ POUR PRÉDIRE UNE RÉPONSE À UN TRAITEMENT PAR PRESSION POSITIVE CONTINUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2014 EP 14382533**

(43) Date of publication of application:
**25.10.2017 Bulletin 2017/43**

(73) Proprietors:
- **Centro de Investigación Biomédica en Red
  28029 Madrid (ES)**
- **Institut de Recerca Biomèdica de Lleida
  Fundació Doctor Pifarré
  25008 Lleida (ES)**
- **Fundación Para la Investigación del Hospital Universitario y Politécnico La Fe de la Comunidad Valenciana
  46026 Valencia (ES)**

(72) Inventors:
- **BARBE ILLA, Eduard Ferran
  25125 Alguaire (ES)**
- **SANCHEZ DE LA TORRE, Manuel
  25110 Alpicat (ES)**
- **GOZAL, David
  Chicago, Illinois 60615 (US)**
- **KHALYFA, Abdelnaby
  Chicago, Illinois 60637 (US)**
- **SANCHEZ DE LA TORRE, Alicia
  25005 Lleida (ES)**
- **MARTINEZ GARCÍA, Miguel Ángel
  46026 Valencia (ES)**

(74) Representative: **Hoffmann Eitle
Hoffmann Eitle S.L.U.
Paseo de la Castellana 140, 3a planta
Edificio LIMA
28046 Madrid (ES)**

(56) References cited:
**WO-A2-2012/083004**

- **XIAOMEI XIE ET AL: "Effects of continuous positive airway pressure therapy on systemic inflammation in obstructive sleep apnea: A meta-analysis", SLEEP MEDICINE, vol. 14, no. 11, 1 November 2013 (2013-11-01), pages 1139-1150, XP055193307, ISSN: 1389-9457, DOI: 10.1016/j.sleep.2013.07.006**
- **GHARIB SINA A ET AL: "Treatment of Obstructive Sleep Apnea Alters Cancer-associated Transcriptional Signatures in Circulating Leukocytes", SLEEP (ROCHESTER), vol. 37, no. 4, April 2014 (2014-04), page 709, XP002755127,**
- **JUNAID MALIK ET AL: "Variables affecting the change in systemic blood pressure in response to nasal CPAP in obstructive sleep apnea patients", SLEEP AND BREATHING ; INTERNATIONAL JOURNAL OF THE SCIENCE AND PRACTICE OF SLEEP MEDICINE, SPRINGER, BERLIN, DE, vol. 12, no. 1, 9 November 2007 (2007-11-09), pages 47-52, XP019582422, ISSN: 1522-1709**

- KASAI T ET AL: "Obstructive Sleep Apnea and Heart Failure", JOURNAL OF THE AMERICAN COLLEGE OF CARDIOLOGY, ELSEVIER, NEW YORK, NY, US, vol. 57, no. 2, 11 January 2011 (2011-01-11), pages 119-127, XP027583089, ISSN: 0735-1097 [retrieved on 2011-01-04]
- MANUEL SÁNCHEZ-DE-LA-TORRE ET AL: "Precision Medicine in Patients With Resistant Hypertension and Obstructive Sleep Apnea", JOURNAL OF THE AMERICAN COLLEGE OF CARDIOLOGY., vol. 66, no. 9, 1 September 2015 (2015-09-01), pages 1023-1032, XP055255463, US ISSN: 0735-1097, DOI: 10.1016/j.jacc.2015.06.1315
- R. DOUG MCEVOY ET AL: "Measuring Blood microRNAs to Provide Personalized Advice to Sleep Apnea Patients With Resistant Hypertension", JOURNAL OF THE AMERICAN COLLEGE OF CARDIOLOGY., vol. 66, no. 9, 1 September 2015 (2015-09-01), pages 1033-1035, XP055255472, US ISSN: 0735-1097, DOI: 10.1016/j.jacc.2015.07.014

## Description

The present invention provides *in vitro* methods to predict the response to treatment with continuous positive airway pressure (CPAP) in patients suffering from obstructive sleep apnea (OSA) and resistant hypertension (RH). The methods have also potential to follow the response of treated patients.

BACKGROUND ART

Continuous positive airway pressure (CPAP) acts as a pneumatic splint to the upper airway during sleep and corrects the obstruction. As a treatment or therapy, CPAP uses mild air pressure to keep an airway open. CPAP is used for people who have breathing problems, such as obstructive sleep apnea (OSA), for which CPAP is the most effective and gold standard treatment, wherein the mild pressure from CPAP prevents the airway from collapsing or becoming blocked. CPAP treatment is an everyday therapy in patients with obstructive sleep apnea. The diagnosis of OSA and the management of patients with OSA, represent an important economic impact in the public health system.

OSA is a highly prevalent and chronic disease considered by Health Authorities as a real problem. It affects 10 % of the middle age old population with an increasing prevalence along age. OSA is characterized by repeating episodes of collapse or obstruction of superior airways during night. These episodes are associated with a worsening of life quality, excessive day somnolence, accidents, blood hypertension, cardiovascular and cerebrovascular diseases, and it is related with a mortality excess due to cardiovascular diseases.

In addition, more than 70 % of patients with resistant hypertension (RH) also suffer from OSA. RH is defined as hypertension that remains above goal blood pressure in spite of using, at once, three antihypertensive agents belonging to different drug classes. As shown by some authors, CPAP treatment leads to significant reductions in 24-h mean blood pressure (BP) (c.f. Martinez-Garcia et al., "Effect of CPAP on Blood Pressure in Patients With Obstructive Sleep Apnea and Resistant Hypertension", Journal of American Medical Association- 2013, Vol. No. 310(22), pp.: 2407-2415).

However, response to CPAP is highly variable and it seems to be not only dependent on adherence to treatment but also it is speculated with some genetic factors. Thus, it has also been observed no response to CPAP treatment though a good adherence exists in some OSA patients.

There aren't nowadays any methods for predicting response to CPAP treatment. On the other side, CPAP treatment is expensive chronic treatment for many patients, including among them those OSA patients, being OSA caused or consequence of any other disease.

Thus, there are documents disclosing some cardiovascular clinical variables or parameters that can be altered during CPAP treatment, such as heart rate variability (c.f. Kufoy et al., "Changes in the Heart Rate variability in Patients with Obstructive Sleep Apnea and Its Response to Acute CPAP Treatment", PlosOne-2012, Vol. No. 7(3), e33769).

Other documents relate to the adherence to treatment being influenced by initial problems of the patients (autotitration), recent life-events, or life mode (alone or with other people), which were associated with a lower CPAP machine use. An example is the document of Lewis et al., "Early predictors of CPAP Use for the Treatment of Obstructive Sleep Apnea", Sleep-2004, Vol. No. 1, pp.: 134-138. Lewis et al., reported that in relation to all initial variables, reporting problems after the first night of CPAP treatments seems the most important predictor of ensuing machine use, and further that a single screening question immediately after autotitration is useful in identifying those at high risk of treatment failure.

Therefore, it is evident that new and better methods to predict response to CPAP treatment are required, improving life quality of those responder patients and avoiding costs and time with the non-responder ones, which can be derived to other alternative therapies, such as surgery, life-style modifications (in particular in obese subjects) and the use of mandibular advancement devices (MAD). In addition, the early detection of patients who will respond adequately to treatment with CPAP, could allow the development of clinical and monitoring specific actions in order to ensure good adherence to CPAP treatment by these patients.

SUMMARY OF THE INVENTION

Inventors have discovered that some microRNA can be used as markers for predicting the response to CPAP treatment in a patient in need of such treatment. The scope of the invention is defined by the appended claims.

MicroRNA (abbreviated miRNA) is a small non-coding RNA molecule (containing about 22 nucleotides) found in plants, animals, and some viruses, which functions in RNA silencing and post-transcriptional regulation of gene expression. Encoded by eukaryotic nuclear DNA in plants and animals and by viral DNA in certain viruses whose genome is based on DNA, miRNAs function via base-pairing with complementary sequences within mRNA molecules.

According to the invention are thus proposed methods involving analysis of the levels of expression of micro-RNAs, which have been detected as differentially expressed between responder and non-responder patients treated with CPAP. A responder patient is one showing a blood pressure (BP) decrease of at least 4.5 mm Hg in relation to initial BP before treatment.

The methods of the invention are reliable methods and they provide high sensitivity and specificity values. In addition, the methods are easily applicable to clinics due to the provision of a quantitative score parameter informing the clinician of the probability of response or not.

In a first aspect the disclosure relates to *in vitro* methods for predicting the response to CPAP treatment, the method comprising determining in an isolated sample of a subject the level of expression of a cardiovascular system functionally related and cardiovascular disease related microRNA, said microRNA selected from the group consisting of miR.100.5p, miR.378a.3p, miR.486.5p, and combinations thereof.

Sequences of all these microRNA may be retrieved from miRbase (www.mirbase.org) Release 21 of June 2014. There can be obtained sequences of *Homo sapiens* and from other indexed species. In particular the accession numbers in miRbase of the herewith referred microRNAs are: miR.378a.3p (MIMAT0000732), miR.100.5p (MIMAT0000098) and miR.486.5p (MIMAT0002177).

The invention proposes thus for the first time the association of the levels of expression of one or more cardiovascular disease-related microRNAs, as well as particular combinations of said microRNAs, for predicting the response to CPAP treatment in a subject that could take benefit from said treatment.

As will be depicted in the examples below, the methods of the invention predict in a highly specific and sensitive way the probability of response to CPAP treatment in a patient (subject) in need thereof. Therefore, responder patients are identified.

Of note is that the microRNAs, which have been detected as differentially expressed between responder and non-responder patients/subjects that are next treated with CPAP, are all microRNA that are also differentially expressed in patients with cardiovascular dysfunction risk or already suffering from any cardiovascular dysfunction in relation to health subjects (subject with no cardiovascular disease or risk of disease). In other words, they are microRNAs most relevant to heart disease or associated with cardiovascular disease. That miRNAs are crucial for the development and proper functioning of the heart has been established and well-accepted by scientific community (see Small et al., "MicroRNA add a new dimension to Cardiovascular Disease", Circulation-2010, Vol. No. 121, pp.: 1022-1032). Thus, for instance miR.100.5p has been associated to cardiomyopathy, wherein it is up-regulated or in higher levels than in health subjects (see Da Costa el at., "MicroRNAs in control of cardiac hypertrophy", Cardiovascular Researh-2012, Vol. No. 93, pp.: 563-572); miR.378a.3p has been associated with the ability to determine efficacy of drugs for cardiac conditions (see WO2012083004); and miR.486.5p has been proposed as marker of cardiovascular disease (see WO2012065113 and Da Costa el at., *supra*), in particular as marker together with other miRNAs of thoracic aortic aneurysm, of diastolic heart failure in the subject, of left ventricular hypertrophy in the absence of diastolic heart failure, of left ventricular remodelling, of ischemia-reperfusion, or a combination of these diseases.

Determining if a subject will be a responder or not to CPAP treatment allows the clinician deciding with much of the crucial information the treatment that should be recommended.

In a second aspect, thus, the disclosure relates also to methods for selecting or recommending initiating CPAP treatment, the method comprising a step of determining in an isolated sample of a subject the level of expression of a cardiovascular system functionally related and cardiovascular disease related microRNA, said microRNA selected from the group consisting of miR.100.5p, miR.378a.3p, miR.486.5p, and combinations thereof, and wherein CPAP treatment is indicated or recommended if the level of expression of any one of miR.100.5p, miR.378a.3p, miR.486.5p is within a range of levels of expression indicative of response to CPAP.

The performance of all these methods may be done using specific means for detecting microRNA expression. Thus, in a third aspect the disclosure relates also to the use of means for detecting expression of a microRNA in an isolated sample of a subject, said means comprising polymerase chain reaction (PCR) reagents, or northern blot reagents, for the prediction of response to continuous positive airway pressure (CPAP) in any of the methods as defined above.

It is also part of the disclosure a kit for detecting the levels of expression of microRNA in an isolated sample of a subject, said kit consisting in means for determining the levels of expression of miR.100.5p, miR.378a.3p, and miR.486.5p.

Further, forms also part of the disclosure the use of the level of expression of a microRNA selected from the group consisting of miR.100.5p, miR.378a.3p, miR.486.5p, and combinations thereof, as a marker for predicting the response to continuous positive airway pressure (CPAP) treatment in a subject in need thereof.

Of note, inventors propose also using these microRNA levels of expression for predicting cardiovascular risk reduction in subjects that will be labelled as responders to CPAP by any of the methods disclosed above. This is so because in a subject that it is likely to be a responder according to its microRNA expression levels before treatment with CPAP, it is also likely that his/her blood pressure will be lowered and so any cardiovascular risk associated to high blood pressure.

Determining if a subject will respond to CPAP before starting the treatment is of great value not only because, as above exposed, the treatment is unfeasible and expensive, but also because CPAP implies many secondary adverse effects or even deleterious effects if adherence to treatment is not the appropriate one. Therefore, if a subject is unlikely to respond, additional complications are avoided if CPAP is excluded from treatment options.

BRIEF DESCRIPTION OF THE DRAWINGS

FIG. 1 (A to C) shows boxplots for miR100.5p levels (as dCt miR100.5p), miR.378a.3p levels (as dCt miR.378a.3p), and miR.486.5p levels (as dCtmiR.486.5p), respectively, in relation with response to CPAP (expressed as ranges of values in X-axis indicating response degrees to CPAP).

FIGs. 2 and 3 are boxplots for, miR100.5p levels (as dCt miR100.5p) and miR.378a.3p levels (as dCt miR.378a.3p), respectively, for non-responders (A) and responders (B) to CPAP.

FIG. 4 depicts a ROC curve of the method for predicting response to CPAP using a model with combined miR.100.5p, miR.378a.3p and miR.486.5p levels from an isolated sample. X-axis shows 1-specificity and Y-axis the sensitivity.

FIG. 5 (A to C) shows boxplots with the change in the mean blood pressure ($\Delta$MBP), measured as the difference of initial MBP minus final MBP, represented in relation with a cut-off point for each dCt miRNA at baseline. Responders represented at right box in each graphic, and non-responders at left box in each graphic. Arrows indicate the median values.

DETAILED DESCRIPTION OF THE INVENTION

All terms as used herein, unless otherwise stated, shall be understood in their ordinary meaning as known in the art. Other more specific definitions for certain terms as used in the present application are as set forth below and are intended to apply uniformly throughout the description and claims unless an otherwise expressly set out definition provides a broader definition.

In the sense of the present invention, the terms "level of expression of microRNA" or "microRNA expression levels" (used interchangeably) encompasses the amount of microRNA (generally expressed as cycle threshold (Ct)) detected in the sample by any means for the analysis of RNA, such as quantitative real-time reverse transcription PCR (qRT-PCR). RT-PCR is used to clone expressed genes by reverse transcribing the RNA of interest into its DNA complement through the use of reverse transcriptase. Subsequently, the newly synthesized cDNA is amplified for the application of quantitative PCR. Compared to other RNA quantification methods, such as northern blot, qRT-PCR is considered to be the most powerful, sensitive, and quantitative assay for the detection of RNA levels. Expression levels of microRNA are usually represented by the cycle threshold (Ct), which is the number of cycles required for a fluorescent signal used in the test to cross a predetermined threshold. Ct is thus the value in which the fluorescent signal exceeds the background level. Ct values are expressed on log scale base 2 and do not have units. Ct for a particular microRNA is thus inversely proportional to the number or miRNA copies in a sample (i.e. to the amount of target nucleic acid). The lower the Ct level the greater the amount of miRNA in the sample. Since the quantification of miRNA expression is influenced by variability in technical conditions as well as by biological variability, normalization of Ct expression levels by subtracting the Ct expression levels of a housekeeping gene is usually performed. Housekeeping genes are genes expressed with low variability among biological samples and thus they are useful for minimizing expression variability. An example of housekeeping gene is the SNORD95 gene that it is known to remain constant throughout the experimental and life conditions and which variability is only influenced by the technical variability (GeneBank accession number of SNORD95 is AY349594, Version 1 of 14.11.2003).

In the sense of the invention the term "cardiovascular disease" or cardiovascular dysfunction" or "cardiovascular dysfunction risk" (used herewith as synonymous) relates to those diseases involving either heart or blood vessels or both that at the end affect the cardiovascular system. Examples include myocardial infarction and myocardial ischaemia, cardiac dysrhythmias, stroke, systemic hypertension (including resistant hypertension), sudden death, and heart failure. The term "cardiovascular-related disease" refers to diseases that may be the cause or consequence of a cardiovascular disease. Among these "cardiovascular-related diseases" are obstructive sleep apnea, the latter also being associated to patients with cardiovascular disease risk or suffering yet any cardiovascular disease.

The "reference control value" means in the context of the present invention, the level or amount of a particular microRNA derived from a group of subjects and allowing classification of a tested sample from a subject into a responder to CPAP group or into a non-responder to CPAP group. Also in the context of the invention the reference control value may be the cut-off allowing classifying a test sample into a group in which CPAP treatment is already taking effect, or in a group in which the CPAP treatment is not yet taking effect. Besides, when in the present invention the expression "out of reference" is employed, it is to be understood that the levels or amount of a particular analysed microRNA is over or below a cut-off determined for said microRNA. The samples may be taken from a subject or group of subjects wherein the presence, absence, stage, or course of a particular response to CPAP has been properly performed previously. This value is used as a threshold to discriminate subjects wherein the condition to be analysed is present from those wherein such condition is absent. Reference control values are usually determined considering similar characteristics of the subjects (age, sex, race, etc.). In addition, the reference control value may be a value from the same subject but measured at different time points, which is the particular case when a method for determining if a CPAP receiving subject is responding to said CPAP treatment. The skilled person in the art, making use of the general knowledge, is able to choose the subject or group of subjects more adequate for obtaining the reference control level for each of the methods of the present invention. Methods for obtaining the reference value from the group of subjects selected are well-known in the state of the art.

Methods for obtaining the reference value from the group of subjects selected are well-known in the state of the art (Burtis C.A. et al., 2008, Chapter 14, section "Statistical Treatment of Reference Values"). In a particular case the reference control value is a cut-off value defined by means of a conventional ROC analysis (Receiver Operating Characteristic analysis).

As above exposed, the disclosure encompasses in vitro methods for predicting the response to continuous positive airway pressure (CPAP) in a subject in need thereof, the method comprising determining in an isolated sample of a subject the level of expression of microRNAs cardiovascular system functionally related and cardiovascular disease related microRNA (microRNAs known to be differentially expressed in patients with cardiovascular dysfunction or with cardiovascular dysfunction risk), said microRNAs selected from the group consisting of miR.100.5p, miR.378a.3p, miR.486.5p, and combinations thereof.

In a particular embodiment of the first aspect of the disclosure optionally in combination with any embodiment below, the response to continuous positive airway pressure is a decrease of blood pressure, expressed as the difference on initial blood pressure minus the final blood pressure after CPAP. In a more particular embodiment, the response to continuous positive airway pressure is a decrease of blood pressure of at least 4.5 mm Hg in relation to initial blood pressure before treatment.

In another more particular embodiment, the response to continuous positive airway pressure is a decrease of blood pressure of at least 4.5 mm Hg in relation to initial blood pressure before treatment and the subject in need thereof is a patient suffering from a cardiovascular disease or cardiovascular-related disease selected from the group consisting of obstructive sleep apnea (OSA), resistant hypertension, and mixtures of these diseases.

In another particular embodiment of the first aspect of the disclosure optionally in combination with any embodiment below, the level of expression of a microRNA of interest is detected by quantitative real-time reverse transcription PCR and it is expressed as a dCt value computed according to formula (I),

$$dCt = Ct\ microRNA - Ct\ housekeeping\ gene\ \ (I),$$

wherein:

- Ct is a cycle threshold, identified as the number of cycles in the quantitative real-time reverse transcription PCR assay required for a fluorescent signal used therein to cross a predetermined threshold;
- . Ct microRNA is the cycle threshold levels of microRNA of interest;
- Ct housekeeping gene denotes the cycle threshold levels of a housekeeping gene; and
- dCt is the difference between the cycle threshold levels of microRNA, on log scale base 2, and the cycle threshold level of a housekeeeping RNA, on log scale base 2.

Therefore, for a particular isolated sample dCt for each miRNA of interest is calculated by means of formula (I) and this value is contrasted with a reference control dCt value.

In a more particular embodiment, the housekeeping gene is the gene coding for an RNA known as the Small nucleolar RNA 95 (SNORD95).

In a particular embodiment of the first aspect of the invention, the levels of expression of miR.100.5p are determined. In yet a more particular embodiment, the levels of expression of miRNA are determined by a dCt value computed according to formula (I), and wherein the dCt of miR.100.5p is lower than a reference control value, it is indicative of response to CPAP.

In a most particular embodiment, the reference control for miR.100.5p is a dCt value of 0.75, and the housekeeping gene is SNORD95.

Univariate analysis of the level of expression of miR.100.5p allows good classification of subjects being good responders from those extremely non-responders. Good responder means that upon receiving CPAP the subject fast responds and BP is lowered. On the other hand, those extremely non-responders are those subjects that upon receiving CPAP a null decrease of BP is observed.

A microRNA amount of miR.100.5p, expressed as dCt(miR.100.5p), higher or lower than 0.75 allows classifying good responder from extremely non-responder to CPAP patients with a ROC area under curve of 91.7 %.

Inventors developed also a model using both the expression levels of miR.378a.3p and of miR.486.5p that allow classifying with high sensitivity and specificity the subjects with all kind of good and bad responses to CPAP. This model allows thus good classification of those responders that perhaps will respond not so fast as a good responder from those non-responders that although not being extremely non-responders are to be clinically labelled as non-responders too.

In a more particular embodiment, the levels of expression of miR.378a.3p and of miR.486.5p are determined by computing for each one a dCt value also according to formula (I). In yet a more particular embodiment the housekeeping gene of formula (I) is SNORD95.

Thus, in a more particular embodiment of the first aspect of the invention, the method comprises determining at least the levels of expression of two miRNA, and:

(i) computing a score of probability (S) of response to CPAP resulting from the sum of individual discrete scores given to the levels of expression of each microRNA; and
(ii) determining that the subject will respond to CPAP if the score of probability is within a range of scores of probability indicative of response.

When more than one miRNA are determined, and in particular the levels of expression of miR.378a.3p and of miR.486.5p, the method of the first aspect of the invention further comprises:

(i) computing a score of probability (S) of response to CPAP resulting from the sum of individual discrete scores, said individual discrete scores given to the levels of expression of each microRNA; and
(ii) determining that the subject will respond to CPAP if the score of probability is within a range of scores of probability indicative of response.

When both of the levels of miR.378a.3p and of miR.486.5p are determined, a high discrimination ability is obtained (AUC = 0.88) between responder and non-responder to CPAP subjects as will be depicted in the examples below. In a more particular embodiment, the levels of miR.378a.3p and of miR.486.5p are determined by quantitative real-time reverse transcription PCR, they are expressed as a dCt value according to formula (I) as defined above; and the score of probability (S) of response to CPAP is computed from the sum of the individual discrete scores given to the levels of expression of miR.378a.3p and of miR.486.5p, wherein said individual discrete scores are given to each of the levels of expression of the microRNA according to the following criteria:

- adding 1 if levels of miR.486.5p, expressed as a dCt value according to formula (I), are higher than a reference control dCt value, most particularly higher than a dCt value of -7.2;
- adding a 2 if levels of miR.378a.3p, expressed as a dCt value according to formula (I), are lower than or equal to a reference control dCt value, most particularly lower than or equal to a dCt value of 2.6;

and wherein a score of probability from 2 to 3 is indicative of response to CPAP.

Particular dCt values of -7.2 and 2.6 are calculated according to formula (I) wherein the housekeeping gene is SNORD95.

Therefore, in a particular embodiment it is proposed firstly determining expression levels of miR.100.5p and if higher discrimination ability is desired further analysis of expression levels of miR.378a.3p and of miR.486.5p is performed.

Precisely in order to improve the sensitivity and specificity of the method of the invention, the inventors propose in another preferred embodiment of the first aspect of the invention, optionally in combination with any embodiments below or above, determining the level of expression of miR.100.5p, miR.378a.3p, and miR.486.5p.

When the levels of expression of the three miR.100.5p, miR.378a.3p, and miR.486.5p are determined, an in order to make the method more feasibly applicable in clinics (easy clinic implementation), the method for the prediction of CPAP response according to the first aspect of the invention comprises:

(i) obtaining a score of probability (S) of response to CPAP, said score of probability being computed from the sum of individual discrete scores given to each of the levels of expression of miR.100.5p, miR.378a.3p, and miR.486.5p; and
(ii) determining that the subject will respond to CPAP if the computed score of probability (S) is within a range of scores of probability indicative of response.

In a more preferred embodiment when a score of probability is computed, the level of expression of miR.100.5p, miR.378a.3p, and miR.486.5p are determined by quantitative real-time reverse transcription PCR; they are expressed as a dCt value according to formula (I) as defined above; and a score of probability (S) of response to CPAP is computed from the sum of the individual discrete scores given to each of the levels of expression of miR.100.5p, of miR.378a.3p and of miR.486.5p, said individual discrete scores given to the levels of expression of each microRNA according to the following criteria:

- adding 1 if levels of miR.100.5p, expressed as a dCt value according to formula (I), are higher than a reference control dCt value;
- adding a 2 if levels of miR.486.5p, expressed as a dCt value according to formula (I), are higher than a reference control dCt value; and
- adding a 3 if levels of miR.378a.3p, expressed as a dCt value according to formula (I), are lower than a reference dCt value;

and wherein a score of probability (S) from 4 to 6 is indicative of response to CPAP.

In particular, a score of probability from 4 to 6 is indicative of response to CPAP in a probability from 84 % to 100 %. A score of probability from 0 to 1 is indicative of non-response to CPAP in a probability from 85.7 % to 96.2%.

In a preferred embodiment, when a score of probability of response to CPAP is calculated, the reference dCt value for the miR.100.5p expression (dCtmiR.100.5p) is 0.4, and a 1 is added to the score when dCtmiR.100.5p is lower than 0.4. In another preferred embodiment, optionally in combination with any embodiment above or below, the reference dCt value for the miR.486.5p expression (dCtmiR.486.5p) is -7.1, and a 2 is added to score when dCtmiR.486.5p is higher than -7.1. In another preferred embodiment, optionally in combination with any embodiment above or below, the reference dCt value for the miR.378a.3p expression (dCt miR.378a.3p) is 2.6, and a 3 is added to score when dCtmiR.486.5p is lower than 2.6.

Particular dCt values of 0.4, -7.1 and 2.6 are calculated according to formula (I) wherein the housekeeping gene is SNORD95.

By using this model including determining the levels of expression of the three miR.100.5p, miR.378a.3p, and miR.486.5p and further obtaining a score of probability of response to CPAP, if the score of probability is from 0 to 1 a probability of response to CPAP treatment is from 3 to 15 %, and if the score of probability is from 2 to 3, a probability of response to CPAP treatment is from 40 % to 80 %.

When the levels of expression of the three miR.100.5p, miR.378a.3p, and miR.486.5p are determined, a high discrimination ability is obtained (ROC AUC = 0.91) between CPAP responder and non-responder subjects, as will be depicted in the examples below. Additionally, this model can be applied to all subject types (extremely good responders, good responders, no-responders, extremely bad responders) and thus, good classification is obtained independently of the degree of response. As will be depicted in the examples below, this model using at least determination of expression of the three miR.100.5p, miR.378a.3p, and miR.486.5p results from a cohort of samples with different response degrees to CPAP, and which was further validated in a separate cohort.

In a particular embodiment of any of the aspects and embodiments of the disclosure the subject is a patient suffering from a cardiovascular disease or cardiovascular-related disease selected from the group consisting of obstructive sleep apnea (OSA), resistant hypertension, myocardial infarction, myocardial ischaemia, cardiac dysrhythmias, stroke, heart failure and mixtures of these diseases. In a more particular embodiment, the subject is a patient suffering from a cardiovascular disease or cardiovascular-related disease selected from the group consisting of obstructive sleep apnea (OSA), resistant hypertension and mixtures of these diseases.

In a most particular embodiment, optionally in combination with any embodiment above or below, the patient is a patient suffering from obstructive sleep apnea, and more particularly a subject suffering from obstructive sleep apnea with resistant hypertension.

In another particular embodiment, optionally in combination with any embodiment above or below, the isolated sample is selected from the group consisting of blood (which can be in turn selected from serum, plasma or whole blood), saliva, a bronchoalveolar lavage and urine.

In another particular embodiment, optionally in combination with any embodiment above or below, the response to CPAP means that blood pressure (BP) decreases <= 4.5 mm Hg (at least 4.5 mm Hg, equivalent to 0.6 kPa) in relation to initial BP before treatment with CPAP.

The disclosure also relates to methods for selecting or recommending initiating CPAP treatment to a patient suffering from a cardiovascular disease or cardiovascular-related disease selected from the group consisting of obstructive sleep apnea (OSA), resistant hypertension, myocardial infarction, myocardial ischaemia, cardiac dysrhythmias, stroke, heart failure and mixtures of these diseases, the methods comprising a step of determining in an isolated sample of a patient the level of expression of a microRNA cardiovascular system functionally related and cardiovascular disease related microRNA, said microRNA selected from the group consisting of miR.100.5p, miR.378a.3p, miR.486.5p, and combinations thereof, and wherein CPAP treatment is indicated or recommended if the level of expression of any one of miR.100.5p, miR.378a.3p, miR.486.5p is within a range of levels of expression indicative of response to CPAP, which means that a response will take place within a high probability, in particular within a probability from 84 % to 100 % .

In a preferred embodiment of these methods for selecting or recommending initiating CPAP treatment, the isolated sample is also selected from the group consisting of blood (which can be in turn selected from serum, plasma or whole blood), saliva, bronchoalveolar lavage and urine. Anyway, a sample from the subject comprising cells or fluids from which microRNA can be isolated is useful.

Among the particular means used for detecting expression of a microRNA in an isolated sample of a subject with the purpose of predicting the response to continuous positive airway pressure (CPAP) in a method as defined above, there can be included the polymerase chain reaction (PCR) with appropriate probes, primers and buffers for detecting any one of miR.100.5p, miR.378a.3p, or miR.486.5p. More particular means are indeed primers for amplification of miR.100.5p, miR.378a.3p, or miR.486.5p, which can be provided separately or in a set of pairs of primers. All these means may form part of a kit or array including all reagents and instructions for detecting said microRNAs. An example of said arrays is the Qiagen® Human Cardiovascular Disease miRNA PCR Array, MIHS-113Z, which profiles the expression of 84 miRNAs, known to exhibit altered expression during cardiovascular disease in relation to subjects not suffering a cardiovascular disease and during development.

Indeed, in a particular embodiment the means for use to carry out the methods of the invention include PCR reagents of those employed in RT-PCR-based techniques, and when coupled, qPCR approaches that can have the advantage of being quantitative. RT-qPCR is commonly used for studying mRNA expression.

It is also part of the disclosure a kit for detecting the levels of expression of microRNA in an isolated sample of a subject, said kit consisting in means for determining the levels of expression of miR.100.5p, miR.378a.3p, and miR.486.5p. In a preferred embodiment the kit comprises primers and probes (optionally labelled with fluorescent emitting molecules) for amplifying and visualizing amplification of miR.100.5p, miR.378a.3p, and miR.486.5p in a PCR.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention.

EXAMPLE

A. Subject features and analysis of microRNA. Statistical analysis of experimental data

For the assessment of the possible contribution of miRNA expression to the prediction of the response to the treatment with CPAP in patients with obstructive sleep apnea (OSA) and resistant hypertension (RH) but without disabling hypersomnia requiring urgent treatment (Epworth sleep scale (ESS) <=18), there were obtained biological samples (plasma collected in tubes with EDTA and anticoagulants) of those patients who were randomized to CPAP in an HIPARCO (See Martinez-Garcia et al., 2013, *supra*).

Response to CPAP is defined as change in mean blood pressure (calculated by the difference initial-final) over 4.5 mmHg.

More in detail, 41 male OSA patients with RH were identified out of a larger prospective interventional trial of patients assessed before and after 3 months of adherent CPAP treatment (>4.5hours/night). Response to CPAP was defined as BP changes >4.5 mmHg. Of the 41 patients, 20 exhibited a reduction in mean BP (mean$\pm$SD, 11.5$\pm$5 mmHg) (Responder Group (RG or B herewith)) and in 21 patients, no significant reductions in mean BP occurred (-1.5$\pm$4.7 mmHg) (Non-responder Group (NRG or A herewith)). miRNAs were isolated from fasting morning plasma of the 41 patients, and expression profiling of cardiovascular system-focused miRNA was performed using custom array (Qiagen) followed by verification with qRT-PCR. A logistic regression model was fitted to identify the miRNAs that predict the favorable BP response. Calibration, discrimination, net reclassification index and cross-validation were assessed.

For the analysis of microRNA levels and correlation with response/non-response to CPAP, the participants were divided into three groups, two groups of 12 patients who were selected for being those with a very good response to CPAP (group B), and those with very low response (extremely non-responders, group A) to CPAP; and a third group with the rest of patients. The groups of 12 members (from A and B) were used as training cohort or as a simplified groups to detect the main differences.

Afterwards, all sample size (n= 41) was used for validation. The obtained model was moreover validated using statistical tests as indicated below.

As above indicated, from each member expression profiling of cardiovascular system-focused miRNA before CPAP treatment was determined with the Qiagen® Human Cardiovascular Disease miRNA PCR Array, MIHS-113Z, which profiles the expression of 84 miRNAs.

Next Table 1 shows the characteristics of 12 membered groups of analysed subjects for simplification:

| | A<br>N=12 | B<br>N=12 | p.overall |
|---|---|---|---|
| Sex: | | | <0.001 |
| Woman | 0 (0.00%) | 0 (0.00%) | |
| Man | 12 (100%) | 12 (100%) | |
| Stroke | 1 (8.33%) | 1 (8.33%) | 1.000 |
| CHD | 4 (33.3%) | 2 (16.7%) | 0.055 |
| Family history | 1 (8.33%) | 1 (8.33%) | 0.186 |
| Diabetes | 5 (41.7%) | 6 (50.0%) | 0.658 |
| Dyslipidemia | 9 (75.0%) | 9 (75.0%) | 0.352 |
| Menopause | 0 (0.00%) | 0 (0.00%) | 0.001 |
| Epworth>=10 | 5 (41.7%) | 4 (33.3%) | 0.925 |
| Previous CVE | 4 (33.3%) | 3 (25.0%) | 0.462 |
| SBP pattern | | | 0.631 |
| Dipper | 3 (25.0%) | 5 (41.7%) | |
| Nondipper | 6 (50.0%) | 5 (41.7%) | |
| Riser | 3 (25.0%) | 2 (16.7%) | |
| Diuretics | 10 (83.3%) | 12 (100%) | 0.233 |
| Number of diuretics | 3.00 [3.00;4.00] | 3.50 [3.00;4.00] | 0.932 |
| Diuretic type | | | 0.425 |
| None | 2 (16.7%) | 0 (0.00%) | |
| Tiazides/D.Xipamides | 5 (41.7%) | 9 (75.0%) | |
| Loop diuretics without<br>Tiazides | 4 (33.3%) | 2 (16.7%) | |
| Pot.Sa/Anti-Aldosterone | 0 (0.00%) | 0 (0.00%) | |
| Tiazides & loop diuretics | 1 (8.33%) | 1 (8.33%) | |
| Mean blood pressure | 113 [107;114] | 116 [111;119] | 0.383 |
| Age | 63.0 [55.0;66.2] | 55.0 [50.0;63.0] | 0.266 |
| Body mass index (BMI) | 31.5 [30.1;34.0] | 32.1 [30.5;37.9] | 0.225 |
| BMI>=30 | 6 (75.0%) | 9 (90.0%) | 0.361 |
| Neck perimeter | 43.0 [42.0;46.0] | 43.5 [41.8;46.0] | 0.286 |
| Epworth | 8.50 [6.75;11.2] | 8.00 [5.00;12.0] | 0.826 |
| Years since diagnosis | 12.5 [12.0;23.0] | 9.00 [4.00;13.5] | 0.096 |
| CRP | 1.35 [0.41;2.52] | 0.88 [0.23;2.40] | 0.446 |
| Glycated hemoglobina | 6.05 [5.57;6.35] | 6.30 [5.55;6.80] | 0.232 |
| Total Cholesterol | 194 [149;218] | 192 [165;209] | 0.944 |
| HDL-cholesterol | 39.0 [32.8;48.0] | 49.0 [35.8;55.0] | 0.436 |
| LDL-cholesterol | 125 [71.0;138] | 108 [80.3;124] | 0.837 |
| AHI | 34.5 [26.0;41.8] | 45.0 [29.5;52.2] | 0.731 |
| AHI>=30 | 8 (66.7%) | 9 (75.0%) | 0.802 |
| CT90 | 6.00 [3.50;10.2] | 7.00 [1.75;30.2] | 0.583 |
| Mean saturation | 93.0 [92.0;94.0] | 90.5 [88.5;92.8] | 0.119 |

Abbreviations: CHD: Coronary heart disease; Epworth: Day somnolence assessed by Epworth test value; SBP: systolic blood pressure; Pot.Sa/Anti-Aldosterone: Postural Orthostatic Tachycardia Syndrome serum aldosterone. CRP: C-reactive protein; AHI: apnea-hypopnea index; CT90: percentage of time with oxygen saturation lower than 90%.

In order to graphically assess linear and non-linear relationships with the change in mean blood pressure, two additional cut-off points were added in base to the median values observed in groups A and B, expanding the intervals of change in mean blood pressure into <=0.5, (0.5,4.5], (4.5,11.5] and >11.5 intervals.

Comparability among groups was assessed by using Mann-Whitney and Kruskal-Wallis test for the comparison of the quantitative characteristics among 2 and 3 groups, respectively, and Fisher test for qualitative variables (c.f.

Hollander M, Wolfe DA (1973) Nonparametric Statistical Methods. New York: John Wiley & Sons Pages 68-75 (two samples), 115-120). A logistic regression model was fitted to predict response to CPAP depending on pretreatment data (c.f. Hosmer DW, Lemeshow S (1989) Applied logistic regression. John Wiley & Sons. Pages 8-10, 58, 60, 85-86). Those variables maximizing the discrimination ability of the model, as measured by AUC, were included in the model provided that had demonstrated their statistically significant contribution according to the Likelihood Ratio test (c.f. Hosmer DW, Lemeshow S (2013) Applied logistic regression. John Wiley & Sons. Pages 12, 14-15, 18, 39-41, 86, 111, 114-115, 125, 231, 261-262, 276, 280, 295, 345, 350, 353). The possible cut-off points for each quantitative variable were selected from the ones dividing the sample into two categories with at least 10, responders or non-responders. The Hosmer-Lemeshow test was used to test model calibration and the continuous net reclassification index was used to decide on the inclusion of those variables without a statistically significant contribution to the multivariate logistic regression model but improving its AUC (c.f. Hosmer DW, Lemeshow S (1989) "Applied logistic regression". John Wiley & Sons, Pages 158-164, 204, 354; and 173-182, 206). The final model was translated into an easy to use score system (sum of integer values). Sensitivity and specificity for each possible cut-off point were estimated. The exact Binomial distribution was used to estimate 95% confidence intervals on proportions. R software and a significance level of 5% were used.

B. Analysis of microRNA levels for prediction purposes.
(prediction of response to CPAP treatment)

For almost all the miRNA, the expression among the 6 higher responders is bigger than the expression of non-responders. The analysis of expression allowed identifying an 8 candidate miRNA subset (miR.100.5p, miR.29a.3p, miR.144.3p, miR.150.5p, miR.7.5p, miR.378a.3p, miR.92a and miR.486.5p). The housekeeping SNORD95 was selected because it showed the lowest variability. This housekeeping was used to estimate dCt for each miRNA according to formula (I)

$$dCt = Ct(miR) - Ct(SNORD95) \ (I)$$

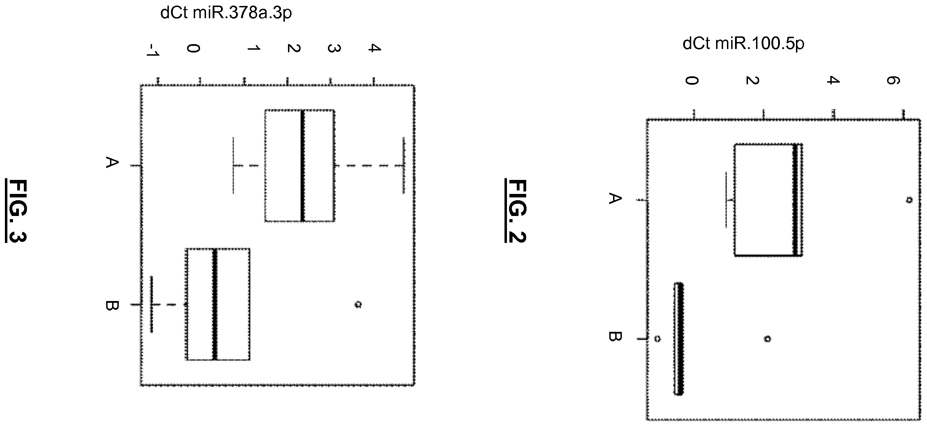

From the analysis of microRNA expression levels in groups A and B of 12 members and further validated within all patients (n= 41), inventors concluded that expression levels of miR.100.5p could discriminate responders from non-responders in case the subjects were extremely good responders and extremely bad responders to CPAP (with a ROC area under curve of 91.7 %, data not shown). This data are derived, for example, from FIG. 2, obtained from the analysis of the values of the 6 most non-responders and the 6 best responders of the 12-membered groups of analysis.

Further, as can be seen from FIGs. 2 and 3 (boxplots for miR100.5p levels and miR.378a.3p levels for non-responders (A) and responders (B)) in the relationship between the response to CPAP and the miRNA expression, there were statistically significant trends in miR.378a.3p apart of in miR.100.5p. Thus, both miRNA showed lower dCt for those patients with higher response to CPAP.

To improve sensitivity and specificity and to get a model or method allowing classification in case of responders and non-responders not being extremely good or bad responders, inventors concluded from the analysis of expression levels of microRNA, that particular combinations of microRNAs were good approaches.

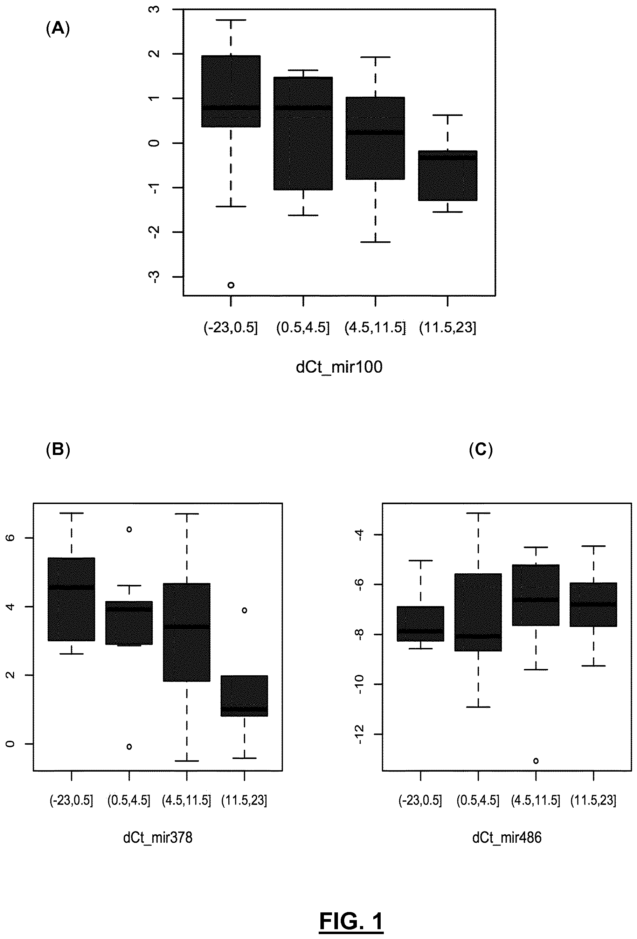

FIG. 1 (panels A to C) shows boxplots for miR100.5p levels (as dCt miR100.5p), miR.378a.3p levels (as dCt miR.378a.3p), and miR.486.5p levels (as dCtmiR.486.5p), respectively, of the 41 isolated samples in relation with response to CPAP (expressed as ranges of values indicating response degrees to CPAP). FIG. 1 clearly denotes that the levels of the three microRNAs viewed together give meaningful information. The two right boxplots of graphics (A) and (B) have median values that are different in a meaningful way to the median values of the two left boxplots of each graphic.

In the training set, there is a statistically significant linear correlation between the change in mean blood pressure and the normalized expression (dCt in reference to housekeeping SNORD95) of miRNA 100.5p and 378a.3p. There is no linear correlation with the normalized expression of miRNA 486.5p. Almost the same result is shown based on the Spearman coefficient. dCt miRNA 100.5p:

Pearson correlation r=-0.49, p-value=0.014
Spearman correlation s= -0.48, p-value = 0.016

dCt miRNA 378a.3p:

Pearson correlation r=-0.56, p-value=0.007
Spearman correlation s= -0.53, p-value = 0.011

dCt miRNA 486.5p:

Pearson correlation r= 0.17, p-value=0.414

Spearman correlation s= 0.19, p-value = 0.387

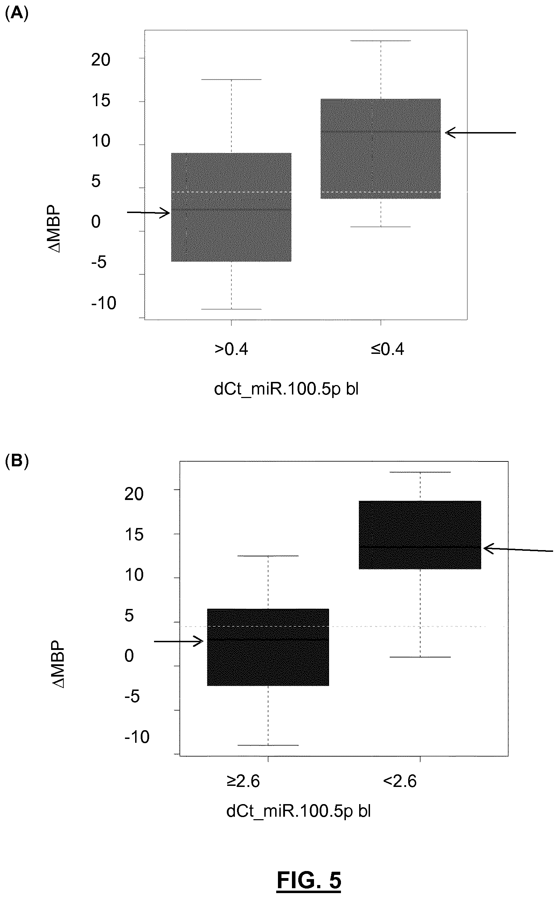

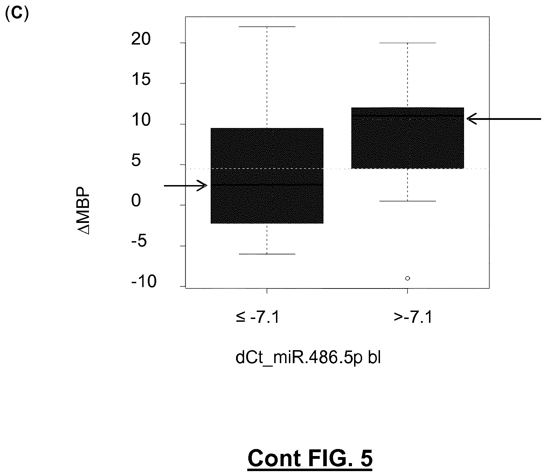

In spite of the absence of a significant linear correlation for dCt miRNA 486.5p, all the cut-off points defined for these miRNA expressions at baseline (before treatment) showed a positive median change in mean blood pressure over 10 mmHg, as illustrated by FIG. 5 (A to C), wherein the boxplot of the change in the mean blood pressure (ΔMBP), with change measured as the difference of initial MBP minus final MBP is represented in relation with each dCt miRNA at baseline for responders and non-responders. It can be seen in any of the graphics, that at baseline the proposed cut-off points in non-responders (left box in each graphic) showed a lower ΔMBP than in responders (right box in each graphic). Arrows indicate the median values.

The advantage of the normalized expression of miRNA 486.5p was that it was not significantly correlated with the normalized expression of neither miRNA 100.5p (r=-0.10, p=0.63) nor miRNA 378a.3p (r=0.05, p-value = 0.84). In contrast, the normalized expressions of dCt miRNA 100.5p and dCt miR 378a.3p are significantly correlated, with r=0.50 and p-value=0.017. The significance of dCt miRNA 486.5p is supported by its significant contribution to the model once adjusted by dCt miRNA 378a.3p (see below).

Inventors determined discriminatory values for each of the miR.378a.3p, miR.486.5p and miR.100.5p. Thus, the expression of miR.378a.3p determined as a dCt value < 2.6, was statistically significant in the univariate model (raw or unadjusted), with p=0.0052. The expression of miR.486.5p, determined as dCt value > -7.1, was also statistically significant in the univariate model, with p=0.0327. Finally, the expression of miR.100.5p, determined as dCt value < 0.4, was also statistically significant in the univariate model, with p=0.0327.

Thus, with the analysis of these three markers, subjects may be separated according to the response level to CPAP.

A model to predict response to CPAP using both the levels of expression of miR.378a.3p and miR.486.5p was elaborated from the data submitted to a fitted logistic regression model (Hosmer DW, Lemeshow S (1989), supra). The model with miR.378a.3p and miR.486.5p used to predict response to CPAP, gave an AUC = 0.88, 95% CI=[0.78,0.98].

Next table 2 shows discretization criteria applied to dCt values of miR.378a.3p and miR.486.5p and the way to compute a score of probability of response (S). Besides, predicted probability of response, as well as associated sensitivity and specificity are listed.

Table 2

| dCt miRNA values | Score of probability (S) | Predicted probability (%) | Sensitivity (%) | Specificity (%) |
|---|---|---|---|---|
| 486<=-7.2 & 378> 2.6 | 0 | 7.30 | 94.4 | 66.7 |
| 486> -7.2 & 378> 2.6 | 1 | 58.76 | 55.5 | 94.4 |
| 486<=-7.2 & 378<=2.6 | 2 | 84.19 | 27.8 | 100.0 |
| 486> -7.2 & 378<=2.6 | 3 | 98.97 | 0.0 | 100.0 |

The score of probability of response (S) from 0 to 3 (i.e. probability of a change in BP > 4.5 mmHg) is computed as:

$$S = 1* + 2**;$$

wherein * means that 1 is added if dCtmiR.486.5p > -7.2; and ** means that 2 is added if dCtmiR.378a.3p <= 2.6, or which is the same a dCtmiR.486.5p > - 7.2 takes the individual discrete score of 1 and it is added to be computed in S; and a dCtmiR.378a.3p <= 2.6 takes the individual discrete score of value 2 and it is added to be computed in S.

If the dCt values of each of miR.486.5p and miR.378a.3p, independently, do not comply with the criteria of being respectively > -7.2, and <= 2.6, no discrete score for that miRNA is added to the formula for computing S.

The Hosmer-Lemeshow test (Table 2) showed no statistically significant lack of fit (p-value= 0.9996). The predicted probability of response to CPAP is almost null (estimated in 0.073) whenever the expression (quantified by dCt) of miR.486.5p is not above -7.2 and the expression of miR.378a.3p is of at least 2.6, meanwhile the probability in the opposite case was estimated in 0.990.

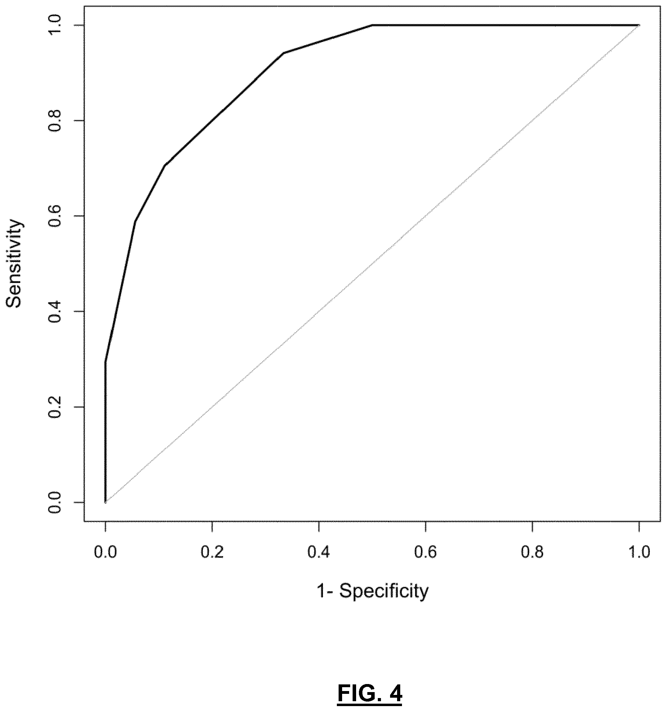

In addition, the miR.100.5p significantly improved the continuous net reclassification index (NRI) in 0.7386, with 95%CI [0.124 - 1.3531] and p-value 0.0185. NRI is estimated as disclosed in Pencina MJ, D'Agostino RB Sr, D'Agostino RB Jr, Vasan RS, "Evaluating the added predictive ability of a new marker: from area under the ROC curve to reclassification and beyond", Stat Med - 2008;27(2):157-72; discussion 207-12. Besides, the addition of miR.100.5p allowed to reach an AUC of 0.91, with 95%CI [0.82, 0.99] and without any significant lack of fit (HL test p-value=0.99). Data using levels of with miR.100.5p, miR.378a.3p and miR.486.5p are depicted in Table 3. Table 3 shows data retrieved from the Hosmer-Lemeshow goodness-of-fit test for the model with miR.100.5p, miR.378a.3p and miR.486.5p. Table 3 shows also relation between score of probability of response to CPAP computable from discretization of dCt values of miR.100.5p, miR.378a.3p and miR.486.5p with the predicted probability of response (in percentage). A ROC curve corresponding to these results can also be seen in FIG. 4.

Table 3:

| Score of probability of response (S) | Predicted probability of response (%) |
|---|---|
| 0 | 3.8 |
| 1 | 14.3 |
| 2 | 46.6 |
| 3 | 78.8 |
| 4 | 84.8 |
| 5 | 96.7 |
| 6 | 99.2 |

Translating the coefficients of the model into a score by adding 1 if dCtmiR.100.5p is <0.4; 2 if dCtmiR.486.5p is >-7.1; and 3 if dCtmiR.378a.3p is <2.6; it could be simplified the use of the model while maintaining exactly the same order of its predicted probabilities.

Thus, the score of probability of response from 0 to 6 (i.e. probability of a change in BP > 4.5 mmHg) in the model with three miRNAs is computed as:

$$S = 1^* + 2^{**} + 3^{***};$$

Wherein * means that 1 is added if dCtmiR.100.5p < 0.4, ** means that 2 is added if dCtmiR.486.5p > -7.1, and *** means that a 3 is added if dCtmiR.378a.3p < = 2.6.

As above for the model with two miRNA, if the dCt values of each of miR.100.5p, miR.486.5p and miR.378a.3p, independently, do not comply with the criteria of being respectively < 0.4, > -7.1, and <= 2.6, no discrete score for that miRNA is added to the formula for computing S.

Table 4 shows the sensitivity and specificity of the decision rule based on each possible cut-off point of the score (S).

Table 4.

| Score of probability of response (S) | Sensitivity (%) | Specificity (%) |
|---|---|---|
| From 1 to 6 | 100.0 | 50.0 |
| From 2 to 6 | 94.1 | 66.7 |
| From 3 to 6 | 70.6 | 88.9 |
| From 4 to 6 | 58.8 | 94.4 |
| From 5 to 6 | 29.4 | 100 |

If the probability of response to CPAP among patients with resistant hypertension is 50 %, by treating only those with Score>0 an expected 50 % of unnecessary treatments could be saved, with no cost since sensitivity is estimated in 100%. Taking into account the uncertainty introduced by the sample size it was estimated a 95% confidence interval (95%CI) for the lack of specificity and sensitivity to be expected in the population by using the exact binomial distribution. Thus, given the specificity of 50% and sensitivity of 100% obtained in the sample, it is expected by applying the score that, with a 95% confidence, between 26% and 74% of unnecessary treatments will be saved, and between 0% and 20% of the patients who would benefit from CPAP treatment will be wrongly untreated.

Since the treatment with CPAP is actually administered to everyone because of its safety and potential benefit for patients with resistant hypertension, reducing the amount of patients unnecessarily treated to 50% (with a 95% confidence interval between 26 and 74%), while keeping a sensitivity of 100% (with a 95% confidence interval between 80 and 100%) when identifying who is and who is not going to benefit from this treatment is a good property of the present invention. Therefore false positive patients (those who initially would be classified as responders and that they aren't at the end) does not represent any drawback, since nowadays all HR patients are treated with CPAP. Therefore if 50 % of the non-useful treatments may be avoided supposes a real advantage.

Data from examples allow concluding that any of miR.100.5p, miR.378a.3p and miR.486.5p levels in an isolated sample of a subject may be used as markers for predicting response to CPAP in a subject in need thereof, with a high sensitivity and specificity. Additionally, particular combinations of these markers improve sensitivity of the method for predicting response to CPAP,or even represent universal methods since allow good classification regardless of the degree of response or no response to CPAP.

It is moreover proposed a method than can easily be applied in clinics, due to the possibility of scoring the levels of the microRNA, thus giving a value indicating or correlating with probability of response. This value may aid the clinician to recommend or not a CPAP treatment avoiding costs and non-effective and uncomfortable situations for the patients.

REFERENCES CITED IN THE APPLICATION

- Martinez-Garcia et al., "Effect of CPAP on Blood Pressure in Patients With Obstructive Sleep Apnea and Resistant Hypertension", Journal of American Medical Association- 2013, Vol. No. 310(22), pp.: 2407-2415.
- Lewis et al., "Early predictors of CPAP Use for the Treatment of Obstructive Sleep Apnea", Sleep-2004, Vol. No. 1, pp.: 134-138
- Small et al., "MicroRNA add a new dimension to Cardiovascular Disease", Circulation-2010, Vol. No. 121, pp.: 1022-1032
- Burtis C.A. et al., 2008, Chapter 14, section "Statistical Treatment of Reference Values.
- WO2012083004
- WO2012065113
- Da Costa el at., "MicroRNAs in control of cardiac hypertrophy", Cardiovascular Researh-2012, Vol. No. 93, pp.: 563-572
- Hollander M, Wolfe DA (1973) Nonparametric Statistical Methods. New York: John Wiley & Sons. Pages 68-75 (two samples), 115-120.
- Hosmer DW, Lemeshow S (1989) Applied logistic regression. John Wiley & Sons. Pages 8-10, 58, 60, 85-86
- Hosmer DW, Lemeshow S (1989) Applied logistic regression. John Wiley & Sons, Pages 158-164, 204, 354; and 173-182, 206.
- Hosmer DW, Lemeshow S (2013) Applied logistic regression. John Wiley & Sons. Pages 12, 14-15, 18, 39-41, 86, 111, 114-115, 125, 231, 261-262, 276, 280, 295, 345, 350, 353.
- Pencina MJ, D'Agostino RB Sr, D'Agostino RB Jr, Vasan RS. Evaluating the added predictive ability of a new marker: from area under the ROC curve to reclassification and beyond. Stat Med. 2008;27(2):157-72; discussion 207-12.

**Claims**

1. An in vitro method for predicting the response to continuous positive airway pressure (CPAP) in a subject with obstructive sleep apnea (OSA) and resistant hypertension, the method comprising determining in an isolated sample of the subject the level of expression of a cardiovascular system functionally related and cardiovascular disease related microRNA, said microRNA selected from the group comprising miR.100.5p or miR.378a.3p, or any combinations thereof, wherein the response is defined in terms of BP (blood pressure), and wherein the response to continuous positive airway pressure is a decrease of mean blood pressure (MBP) of at least 4.5 mm Hg in relation to initial mean blood pressure before treatment.

2. The method according to claims 1, wherein the level of expression of microRNA is detected by quantitative real-time reverse transcription PCR and it is expressed as a dCt value computed according to formula (I),

$$dCt = Ct\ microRNA - Ct\ housekeeping\ gene\ (I),$$

wherein:

- Ct is a cycle threshold, identified as the number of cycles in the quantitative real-time reverse transcription PCR assay required for a fluorescent signal used therein to cross a predetermined threshold;
- Ct microRNA is the cycle threshold levels of microRNA of interest;
- Ct housekeeping gene denotes the cycle threshold levels of a housekeeping gene; and
- dCt is the difference between the cycle threshold levels of microRNA, on log scale base 2, and the cycle threshold level of a housekeeeping RNA, on log scale base 2.

3. The method according to any one of claims 1 to 2, which comprises determining the level of expression of miR.100.5p.

4. The method according to any one of claims 1 to 2, which comprises determining the level of expression of miR.378a.3p and the level of expression of miR.486.5p.

5. The method according to claim 4, comprising determining the level of expression of miR.100.5p, miR.378a.3p, and miR.486.5p.

6. The method according to any one of claims 1 to 4, wherein at least the levels of expression of two miRNA are determined, and the method comprises:

    a. computing a score of probability (S) of response to CPAP resulting from the sum of individual discrete scores given to the levels of expression of each microRNA; and
    b. determining that the subject will respond to CPAP if the score of probability is within a range of scores of probability indicative of response.

7. The method according to claim 6, wherein the levels of expression of miR.378a.3p and of miR.486.5p are determined by quantitative real-time reverse transcription PCR and they are expressed as a dCt value according to formula (I) as defined in claim 2; and a score of probability (S) of response to CPAP is computed from the sum of the individual discrete scores given to the levels of expression of miR.378a.3p and of miR.486.5p, said individual discrete scores given to the levels of expression of each microRNA according to the following criteria:

    a. adding 1 if levels of miR.486.5p, expressed as a dCt value according to formula (I), are higher than a reference control dCt value;
    b. adding a 2 if levels of miR.378a.3p, expressed as a dCt value according to formula (I), are lower than or equal to a reference control dCt value;

and wherein a score of probability (S) from 2 to 3 is indicative of response to CPAP.

8. The method according to claim 7, wherein the reference control value for the levels of miR.486.5p is a dCt value of -7.2 and the reference control value for the levels of miR.378a.3p is a dCt value of 2.6, being the dCt values calculated according to formula (I) wherein the housekeeping gene is SNORD 95.

9. The method according to claim 6, wherein the levels of expression of miR.100.5p, of miR.378a.3p and of miR.486.5p are determined by quantitative real-time reverse transcription PCR and they are expressed as a dCt value according to formula (I) as defined in claim 2; and a score of probability (S) of response to CPAP is computed from the sum of the individual discrete scores given to the levels of expression of miR.100.5p , of miR.378a.3p and of miR.486.5p, said individual discrete scores given to the levels of expression of each microRNA according to the following criteria:

    a. adding 1 if levels of miR.100.5p, expressed as a dCt value according to formula (I), are lower than a reference control value;
    b. adding a 2 if levels of miR.486.5p, expressed as a dCt value according to formula (I), are higher than a reference control value; and
    c. adding a 3 if levels of miR.378a.3p, expressed as a dCt value according to formula (I), are lower than a reference value;

and wherein a score of probability (S) from 4 to 6 is indicative of response to CPAP.

10. The method according to claim 9, wherein the reference control value for the levels of miR.100.5p is a dCt value of 0.4; the reference control value for the levels of miR.486.5p is a dCt value of -7.1 and the reference control value for the levels of miR.378a.3p is a dCt value of 2.6, being the dCt values calculated according to formula (I) wherein the housekeeping gene is SNORD 95.

11. The method according to any one of claims 1 to 10, wherein the isolated sample is selected from the group consisting of blood, saliva, bronchoalveolar lavage and urine.

12. Use of means for detecting expression of a microRNA in an isolated sample of a subject, said means selected from the group consisting of polymerase chain reaction (PCR) reagents and/or northern blot reagents for predicting the response to continuous positive airway pressure (CPAP) treatment in a method as defined in any one of claims 1 to 11.

**Patentansprüche**

1. In-Vitro-Verfahren zur Vorhersage der Reaktion auf einen kontinuierlichen positiven Atemwegdruck (CPAP) in einem Individuum mit obstruktiver Schlafapnoe (OSA) und resistentem Bluthochdruck, wobei das Verfahren das Bestimmen in einer isolierten Probe des Individuums des Expressionsniveaus einer mit dem Herz-Kreislauf-System funktionell in Beziehung stehenden und mit einer Herz-Kreislauf-Erkrankung in Beziehung stehenden mikroRNA umfasst, wobei die genannte mikroRNA aus der Gruppe umfassend miR.100.5p oder miR.378a.3p, und Kombinationen derselben ausgewählt wird, wobei die Reaktion in Form von BP (Blutdruck) definiert wird, und wobei die Reaktion auf einen kontinuierlichen positiven Atemwegdruck eine Senkung des Mittelblutdrucks (MBP) von mindestens 4,5 mm Hg in Bezug auf den anfänglichen Mittelblutdruck vor der Behandlung ist.

2. Verfahren nach Anspruch 1, wobei das Expressionsniveau von mikroRNA mittels quantitativer Echtzeit-Reverse-Transkriptase-PCR erfasst wird und es als dCt-Wert ausgedrückt wird, berechnet gemäß der Formel (I),

$$dCt= Ct\ mikroRNA - Ct\ Haushaltsgen\ (I),$$

wobei:

- Ct eine Zyklusschwelle ist, identifiziert als die Anzahl von Zyklen im quantitativen Echtzeit-Reverse-Transkriptase-PCR-Versuch, welche für ein darin verwendetes Fluoreszenzsignal benötigt wird, um eine vorbestimmte Schwelle zu überschreiten;
- Ct mikroRNA die Zyklusschwellenniveaus der mikroRNA von Interesse ist;
- Ct Haushaltsgen die Zyklusschwellenniveaus eines Haushaltsgens bezeichnet; und
- dCt der Unterschied zwischen den Zyklusschwellenniveaus der mikroRNA, auf logarithmischer Skala mit Basis 2, und dem Zyklusschwellenniveau einer Haushalts-RNA, auf logarithmischer Skala mit Basis 2, ist.

3. Verfahren nach einem der Ansprüche 1 bis 2, welches das Bestimmen des Expressionsniveaus von miR.100.5p umfasst.

4. Verfahren nach einem der Ansprüche 1 bis 2, welches das Bestimmen des Expressionsniveaus von miR.378a.3p und des Expressionsniveaus von miR.486.5p umfasst.

5. Verfahren nach Anspruch 4, welches das Bestimmen des Expressionsniveaus von miR.100.5p, miR.378a.3p und miR.486.5p umfasst.

6. Verfahren nach einem der Ansprüche 1 bis 4, wobei mindestens die Expressionsniveaus von zwei miRNA bestimmt werden, und das Verfahren Folgendes umfasst:

a. das Berechnen eines Wahrscheinlichkeitswertes (S) der Reaktion auf CPAP, welcher sich aus der Summe von einzelnen eigenständigen Werten ergibt, welche den Expressionsniveaus jeder mikroRNA erteilt werden; und
b. das Bestimmen, dass das Individuum auf CPAP reagieren wird, wenn der Wahrscheinlichkeitswert innerhalb eines Bereichs von Wahrscheinlichkeitswerten liegt, welche auf eine Reaktion hindeuten.

7. Verfahren nach Anspruch 6, wobei die Expressionsniveaus von miR.378a.3p und von miR.486.5p mittels quantitativer Echtzeit-Reverse-Transkriptase-PCR bestimmt werden und sie als dCt-Wert gemäß der Formel (I), wie in Anspruch 2 definiert, ausgedrückt werden; und ein Wahrscheinlichkeitswert (S) der Reaktion auf CPAP aus der Summe von den einzelnen eigenständigen Werten berechnet wird, welche den Expressionsniveaus von miR.378a.3p und von miR.486.5p erteilt werden, wobei die genannten einzelnen eigenständigen Werte, welche den Expressionsniveaus jeder mikroRNA erteilt werden, gemäß der folgenden Kriterien sind:

    a. es wird 1 hinzugefügt, wenn die Niveaus von miR.486.5p, ausgedrückt als dCt-Wert gemäß der Formel (I), höher als ein dCt-Referenzkontrollwert sind;
    b. es wird 2 hinzugefügt, wenn die Niveaus von miR.378a.3p, ausgedrückt als dCt-Wert gemäß der Formel (I), kleiner oder gleich als ein dCt-Referenzkontrollwert sind;

und wobei ein Wahrscheinlichkeitswert (S) von 2 bis 3 auf die Reaktion auf CPAP hindeutet.

8.  Verfahren nach Anspruch 7, wobei der Referenzkontrollwert für die Niveaus von miR.486.5p ein dCt-Wert von -7,2 ist und der Referenzkontrollwert für die Niveaus von miR.378a.3p ein dCt-Wert von 2,6 ist, wobei die dCt-Werte gemäß der Formel (I) berechnet werden, wobei das Haushaltsgen SNORD 95 ist.

9.  Verfahren nach Anspruch 6, wobei die Expressionsniveaus von miR.100.5p, von miR.378a.3p und von miR.486.5p mittels quantitativer Echtzeit-Reverse-Transkriptase-PCR bestimmt werden und sie als dCt-Wert gemäß der Formel (I), wie in Anspruch 2 definiert, ausgedrückt werden; und ein Wahrscheinlichkeitswert (S) der Reaktion auf CPAP aus der Summe von den einzelnen eigenständigen Werten berechnet wird, welche den Expressionsniveaus von miR.100.5p, von miR.378a.3p und von miR.486.5p erteilt werden, wobei die genannten einzelnen eigenständigen Werten, welche den Expressionsniveaus jeder mikroRNA erteilt werden, gemäß der folgenden Kriterien sind:

    a. es wird 1 hinzugefügt, wenn die Niveaus von miR.100.5p, ausgedrückt als dCt-Wert gemäß der Formel (I), kleiner als ein Referenzkontrollwert sind;
    b. es wird 2 hinzugefügt, wenn die Niveaus von miR.486.5p, ausgedrückt als dCt-Wert gemäß der Formel (I), höher als ein Referenzkontrollwert sind; und
    c. es wird 3 hinzugefügt, wenn die Niveaus von miR.378a.3p, ausgedrückt als dCt-Wert gemäß der Formel (I), kleiner als ein Referenzwert sind; und

wobei ein Wahrscheinlichkeitswert (S) von 4 bis 6 auf die Reaktion auf CPAP hindeutet.

10. Verfahren nach Anspruch 9, wobei der Referenzkontrollwert für die Niveaus von miR.100.5p ein dCt-Wert von 0,4 ist; der Referenzkontrollwert für die Niveaus von miR.486.5p ein dCt-Wert von -7,1 ist und der Referenzkontrollwert für die Niveaus von miR.378a.3p ein dCt-Wert von 2,6 ist, wobei die dCt-Werte gemäß der Formel (I) berechnet werden, wobei das Haushaltsgen SNORD 95 ist.

11. Verfahren nach einem der Ansprüche 1 bis 10, wobei die isolierte Probe aus der Gruppe bestehend aus Blut, Speichel, bronchoalveoläre Spülung und Urin ausgewählt wird.

12. Verwendung von Mittels zur Erfassung der Expression einer mikroRNA in einer isolierten Probe eines Individuums, wobei die genannten Mittel aus der Gruppe bestehend aus Reagenzien der Polymerase-Kettenreaktion (PCR) und/oder Reagenzien des Northern-Blots zur Vorhersage der Reaktion auf eine kontinuierliche positive Atemwegdruck (CPAP)-Behandlung in einem Verfahren wie in einer der Ansprüche 1 bis 11 definiert ausgewählt werden.

**Revendications**

1.  Procédé *in vitro* pour prédire une réponse à ventilation à pression positive continue (CPAP) chez un sujet atteint d'apnée obstructive du sommeil (AOS) et hypertension réfractaire, le procédé comprenant la détermination dans un échantillon isolé du sujet du niveau d'expression d'un microARN lié fonctionnellement au système cardiovasculaire et lié à maladie cardiovasculaire, ledit microARN étant choisi parmi le groupe comprenant miR.100.5p ou miR.378a.3p, ou n'importe quelle combinaison de ceux-ci, dans lequel la réponse est définie en termes de TA (tension artérielle), et dans lequel la réponse à ventilation à pression positive continue est une réduction de la tension artérielle moyenne (TAM) d'au moins 4,5 mm Hg par rapport à la tension artérielle moyenne initiale avant le traitement.

2.  Procédé selon la revendication 1, dans lequel le niveau d'expression de microARN est détecté par PCR de transcription inverse quantitative en temps réel et il est exprimé comme une valeur dCt calculée selon la formule (I),

$$dCt = Ct\ microARN - Ct\ gène\ domestique\ (I),$$

dans laquelle

- Ct est un seuil de cycle, identifié comme le nombre de cycles dans l'essai de PCR de transcription inverse quantitative en temps réel requis pour qu'un signal fluorescent utilisé en son sein traverse un seuil prédéterminé ;
- Ct microARN est les niveaux de seuil de cycle de microARN d'intérêt ;
- Ct gène domestique dénote les niveaux de seuil de cycle d'un gène domestique ; et
- dCt est la différence entre les niveaux de seuil de cycle de microARN, sur la base d'échelle logarithmique 2, et le niveau de seuil de cycle d'un ARN de gène domestique, sur la base d'échelle logarithmique 2.

3. Procédé selon l'une quelconque des revendications 1 ou 2, qui comprend la détermination du niveau d'expression de miR.100.5p.

4. Procédé selon l'une quelconque des revendications 1 ou 2, qui comprend la détermination du niveau d'expression de miR.378a.3p et le niveau d'expression de miR.486.5p.

5. Procédé selon la revendication 4, qui comprend la détermination du niveau d'expression de miR.100.5p, miR.378a.3p et miR.486.5p.

6. Procédé selon l'une quelconque des revendications 1 à 4, dans lequel au moins les niveaux d'expression de deux miARN sont déterminés, et le procédé comprend :

   a. calculer une cote de probabilité (S) de réponse à CPAP résultant de l'addition de cotes discrètes individuelles données aux niveaux d'expression de chaque microARN ; et
   b. déterminer que le sujet répondra à CPAP si la cote de probabilité se trouve dans un intervalle de cotes de probabilité indicatives de réponse.

7. Procédé selon la revendication 6, dans lequel les niveaux d'expression de miR.378a.3p et de miR.486.5p sont déterminés par PCR de transcription inverse quantitative en temps réel et ils sont exprimés comme une valeur dCt selon la formule (I) telle que définie dans la revendication 2 ; et une cote de probabilité (S) de réponse à CPAP est calculée à partir de l'addition des cotes discrètes individuelles données aux niveaux d'expression de miR.378a.3p et de miR.486.5p, lesdites cotes discrètes individuelles étant données aux niveaux d'expression de chaque microARN selon les critères suivants :

   a. ajouter 1 si les niveaux de miR.486.5p, exprimés comme une valeur dCt selon la formule (I), sont supérieurs à une valeur dCt de contrôle de référence ;
   b. ajouter 2 si les niveaux de miR.378a.3p, exprimés comme une valeur dCt selon la formule (I), sont inférieurs ou égaux à une valeur dCt de contrôle de référence ;

   et dans lequel une cote de probabilité (S) de 2 à 3 est indicative de réponse à CPAP.

8. Procédé selon la revendication 7, dans lequel la valeur de contrôle de référence pour les niveaux de miR.486.5p est une valeur dCt de -7,2 et la valeur de contrôle de référence pour les niveaux de miR.378a.3p est une valeur dCt de 2,6, les valeurs dCt étant calculées selon la formule (I) dans laquelle le gène domestique est SNORD 95.

9. Procédé selon la revendication 6, dans lequel les niveaux d'expression de miR.100.5p, de miR.378a.3p et de miR.486.5p sont déterminés par PCR de transcription inverse quantitative en temps réel et ils sont exprimés comme une valeur dCt selon la formule (I) telle que définie dans la revendication 2 ; et une cote de probabilité (S) de réponse à CPAP est calculée à partir de l'addition des cotes discrètes individuelles données aux niveaux d'expression de miR.100.5p, de miR.378a.3p et de miR.486.5p, lesdites cotes discrètes individuelles étant données aux niveaux d'expression de chaque microARN selon les critères suivants :

   a. ajouter 1 si les niveaux de miR.100.5p, exprimés comme une valeur dCt selon la formule (I), sont inférieurs à une valeur de contrôle de référence ;
   b. ajouter 2 si les niveaux de miR.486.5p, exprimés comme une valeur dCt selon la formule (I), sont supérieurs à une valeur de contrôle de référence ;

c. ajouter 3 si les niveaux de miR.378a.3p, exprimés comme une valeur dCt selon la formule (I), sont inférieurs à une valeur dCt de contrôle de référence ;

et dans lequel une cote de probabilité (S) de 4 à 6 est indicative de réponse à CPAP.

10. Procédé selon la revendication 9, dans lequel la valeur de contrôle de référence pour les niveaux de miR.100.5p. est une valeur dCt de 0,4 ; la valeur de contrôle de référence pour les niveaux de miR.486.5p. est une valeur dCt de -7,1 et la valeur de contrôle de référence pour les niveaux de miR.378a.3p. est une valeur dCt de 2,6, les valeurs dCt étant calculées selon la formule (I) dans laquelle le gène domestique est SNORD 95.

11. Procédé selon l'une quelconque des revendications 1 à 10, dans lequel l'échantillon isolé est choisi parmi le groupe composé de sang, salive, lavage bronchoalvéolaire et urine.

12. Utilisation de moyens pour détecter l'expression d'un microARN dans un échantillon isolée d'un sujet, lesdits moyens étant choisis parmi le groupe composé de réactifs de réaction en chaîne de la polymérase (PCR) et/ou des réactifs de transfert Northern pour prédire la réponse au traitement par ventilation à pression positive continue (CPAP) dans un procédé tel que défini dans l'une quelconque des revendications 1 à 11.

**FIG. 1**

**FIG. 2**

**FIG. 3**

**FIG. 4**

**(A)**

**(B)**

**FIG. 5**

**(C)**

Cont FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012083004 A

- WO 2012065113 A

### Non-patent literature cited in the description

- **MARTINEZ-GARCIA et al.** Effect of CPAP on Blood Pressure in Patients With Obstructive Sleep Apnea and Resistant Hypertension. *Journal of American Medical Association,* 2013, vol. 310 (22), 2407-2415
- **KUFOY et al.** Changes in the Heart Rate variability in Patients with Obstructive Sleep Apnea and Its Response to Acute CPAP Treatment. *PlosOne,* 2012, vol. 7 (3), e33769
- **LEWIS et al.** Early predictors of CPAP Use for the Treatment of Obstructive Sleep Apnea. *Sleep,* 2004, 134-138
- **SMALL et al.** MicroRNA add a new dimension to Cardiovascular Disease. *Circulation,* 2010, 1022-1032
- **DA COSTA.** MicroRNAs in control of cardiac hypertrophy. *Cardiovascular Researh,* 2012, 563-572
- **BURTIS C.A. et al.** Statistical Treatment of Reference Values. 2008
- **HOLLANDER M ; WOLFE DA.** Nonparametric Statistical Methods. John Wiley & Sons, 1973, 68-75

- **HOSMER DW ; LEMESHOW S.** Applied logistic regression. John Wiley & Sons, 1989, 8-10, 58, , 60, , 85-86
- **HOSMER DW ; LEMESHOW S.** Applied logistic regression. John Wiley & Sons, 2013, 12, , 14-15, 18, , 39-41, 86, , 111, , 114-115, 125, , 231, , 261-262
- **HOSMER DW ; LEMESHOW S.** Applied logistic regression. John Wiley & Sons, 1989, 158-164, 204, , 354, , 173-182, 206
- **PENCINA MJ ; D'AGOSTINO RB SR ; D'AGOSTINO RB JR ; VASAN RS.** Evaluating the added predictive ability of a new marker: from area under the ROC curve to reclassification and beyond. *Stat Med,* 2008, vol. 27 (2), 157-72
- 12, 14-15, 18, 39-41, 86, 111, 114-115, 125,. **HOSMER DW ; LEMESHOW S.** Applied logistic regression. John Wiley & Sons, 2013
- **PENCINA MJ ; D'AGOSTINO RB SR ; D'AGOSTINO RB JR ; VASAN RS.** Evaluating the added predictive ability of a new marker: from area under the ROC curve to reclassification and beyond. *Stat Med.,* 2008, vol. 27 (2), 157-72